(19) Europäisches Patentamt
European Patent Office
Office européen des brevets

(11) Veröffentlichungsnummer: **0 440 148 A1**

(12) # EUROPÄISCHE PATENTANMELDUNG

(21) Anmeldenummer: **91101097.3**

(51) Int. Cl.5: **A61K 31/66**, C07F 9/40

(22) Anmeldetag: **28.01.91**

(30) Priorität: **02.02.90 DE 4003054**

(43) Veröffentlichungstag der Anmeldung:
**07.08.91 Patentblatt 91/32**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Peyman, Anuschirwan, Dr.**
**14 Summer Road**
**Brookline, MA 02146(US)**
Erfinder: **Uhlmann, Eugen, Dr.**
**Zum Talblick 31**
**W-6246 Glashütten/Taunus(DE)**
Erfinder: **Budt, Karlheinz, Dr.**
**Am Flachsland 18**
**W-6233 kelkheim/Taunus(DE)**
Erfinder: **Knolle, Jochen, Dr.**
**Höchsster Strasse 21**
**W-6239 Krifel(DE)**
Erfinder: **Winkler, Irvin, Dr.**
**In den Eichen 40**
**W-6237 Liederbach(DE)**
Erfinder: **Helsberg, Matthias, Dr.**
**Münsterer Strasse 14**
**W-6233 kelkheim/Taunus(DE)**

(54) **Verwendung von Benzylphosphonsäurederivaten zur Behandlung von durch Viren verursachte Krankheiten.**

(57) Die Verwendung der Verbindung der Formel I,

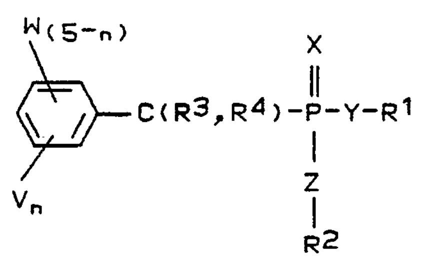

I

in der V für Alkylgruppe, Fluor, Chlor, Brom oder Jod steht, n für eine ganze Zahl von 1 bis 5, u für Alkyl-, Alkenyl-, Alkinyl-, Alkoxygruppe, Cyanid, Nitro, Carboxy, Wasserstoff, Cycloalkyl-, Aryl-, Aralkyl- oder Carboalkoxygruppe steht, $R^1$ und $R^2$ für Alkyl-, Alkenyl-, Alkinyl-, Cycloalkyl- oder Halogenalkylgruppe, Natrium, Kalium, Calcium, Magnesium, Aluminium, Lithium, Ammonium, Triethylammonium oder Wasserstoff stehen oder $R^1$ und $R^2$ zusammen einen cyclischen Diester bilden, $R^3$ und $R^4$ für Alkyl-, Alkenyl-, Alkinyl-, Carboalkyloxy-, Cycloalkyl-oder Alkoxygruppe, Wasserstoff, Fluor, Chlor, Brom oder Jod stehen, X, Y und Z für Sauerstoff oder Schwefel stehen, zur Behandlung von Erkrankungen, die durch DNA- oder RNA-Viren hervorgerufen werden, wird beschrieben.

EP 0 440 148 A1

# VERWENDUNG VON BENZYLPHOSPHONSÄUREDERIVATEN ZUR BEHANDLUNG VON DURCH VIREN VERURSACHTE KRANKHEITEN

Benzylphosphonsäuren wurden bereits beschrieben. Entsprechend den Angaben in der Literatur finden Benzylphosphonsäuren Verwendung als Zwischenprodukte für die Herstellung von optischen Aufhellern oder als Flammschutzmittel (DOS 3001065; J. Chin. Chem. Soc., 31, 1984, Seite 311; J. Polymer Sci., Part A, 27, 1989, Seite 2239).

Benzylphosphonsäurederivate sind nicht als wirksame antivirale Verbindungen beschrieben worden (J.C.H. Mao et al., Antimicrob. Agents Chemother. 27, 1985, Seite 197).

Überraschend wurde nun gefunden, daß Halogenbenzylphosphonsäurederivate oder Alkylbenzylphosphonsäurederivate antivirale Aktivität aufweisen.

Die Erfindung betrifft daher:

1. Verbindung der Formel I,

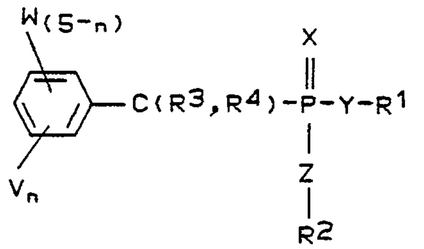

I

in der

V

für eine geradkettige oder verzweigte Alkylgruppe mit 1 bis 4 Kohlenstoffatomen, Fluor, Chlor, Brom, Jod oder Wasserstoff steht,

n

für eine ganze Zahl von 1 bis 5 steht,

W

für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine geradkettige oder verzweigte Alkoxygruppe mit 1 bis 10 Kohlenstoffatomen, eine Arylgruppe mit 6, 10 oder 14 Kohlenstoffatomen, eine Alkylgruppe mit 1 bis 4 Kohlenstoffatomen, ein oder mehrfach substituiert durch den Rest der Formel

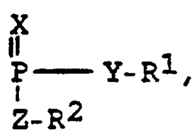

den Rest der Formel $-COOR^6$ oder Halogen, in der Halogen Chlor, Brom, Jod oder Fluor bedeutet, einen ankondensierten Aromaten, der durch eine Alkylgruppe mit 1 bis 4 Kohlenstoffatomen substituiert sein kann, Cyanid, Nitro oder Wasserstoff steht oder für den Rest der Formel Ia steht,

$-COOR^6$ Ia

wobei

$R^6$

eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff oder eine geradkettige oder verzweigte Halogenalkylgruppe mit 1 bis 20 Kohlenstoffatomen bedeutet, in der Halogen Chlor,

Brom, Jod oder Fluor bedeutet,

$R^1$ und $R^2$,

die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff, eine geradkettige oder verzweigte Halogenalkylgruppe mit 1 bis 20 Kohlenstoffatomen stehen, in der Halogen Chlor, Brom, Jod oder Fluor bedeutet, oder $R^1$ und $R^2$ zusammen einen cyclischen Diester mit 2 bis 6 Kohlenstoffatomen im Ring bilden,

$R^3$ und $R^4$,

die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Aralkylgruppe mit 7 bis 20 Kohlenstoffatomen, eine geradkettige oder verzweigte Alkoxygruppe mit 1 bis 10 Kohlenstoffatomen, Wasserstoff, Fluor, Chlor, Brom oder Jod stehen oder für den Rest der Formel Ib stehen,

$-COOR^7$ Ib

wobei

$R^7$

eine geradkettige oder verzweigte Alkylgruppe mit 1 bis 4 Kohlenstoffatomen bedeutet und

X, Y und Z,

die gleich oder verschieden sein können, für Sauerstoff oder Schwefel stehen,

als Arzneimittel.

2. Die Verwendung der Verbindung der Formel I zur Herstellung eines Arzneimittels zur Behandlung von Erkrankungen, die durch DNA- oder RNA-Viren hervorgerufen werden.

Unter der Bezeichnung Alkylgruppe mit 1 bis 10 Kohlenstoffatomen sind beispielsweise folgende Reste zu verstehen: Methyl, Ethyl, Propyl, Isopropyl, n-Butyl, sec.-Butyl, tert.-Butyl, 2,2-Dimethyl-1-propyl, n-Pentyl, n-Hexyl, n-Heptyl, n-Octyl, n-Nonyl und n-Decyl. Mit der Bezeichnung Alkenylgruppe mit 2 bis 10 Kohlenstoffatomen sind z.B. folgende Verbindungen gemeint: Ethenyl, Propenyl, Butenyl, n-Pentenyl, n-Hexenyl, n-Heptenyl, n-Octenyl, n-Nonenyl oder n-Decenyl. Mit der Bezeichnung Alkinylgruppe mit 2 bis 10 Kohlenstoffatmen sind z.B. folgende Verbindungen gemeint: Ethinyl, Propinyl, Butinyl, n-Pentinyl, n-Hexinyl, n-Heptinyl, n-Noninyl, n-Octinyl oder n-Decinyl. Unter einer Aralkylgruppe mit 7 bis 16 Kohlenstoffatomen sind z.B. folgende Reste zu verstehen: Phenylmethyl, Phenylethyl, Phenylbutyl, Phenylpropyl, Phenylpentyl, Phenylhexyl, Phenylheptyl, Phenyloctyl, Phenylnonyl oder Phenyldecyl. Unter einer Cycloalkylgruppe mit 3 bis 8 Kohlenstoffatomen sind Reste zu verstehen wie Cyclopropyl, Cyclobutyl, Cyclopentyl, Cyclohexyl, Cycloheptyl oder Cyclooctyl. Alkoxygruppen mit 1 bis 4 Kohlenstoffatomen sind beispielsweise Reste wie Methoxy, Ethoxy, Propoxy, Isopropoxy, n-Butoxy, sec.-Butoxy oder tert.-Butoxy. Arylgruppen mit 6, 10 oder 14 Kohlenstoffatomen sind z.B. Reste wie Phenyl, Naphthyl, Anthryl oder Phenanthryl. Ankondensierte Aromaten sind z.B. Reste die zusammen mit der Phenylgruppe der Formel I Reste wie Naphthyl, Anthryl oder Phenanthryl bilden.

Eine Verbindung der Formel I, in der

V

für Brom, Wasserstoff oder Methyl steht,

n

für eine ganze Zahl von 1 bis 5 steht,

W

für Methoxy oder Wasserstoff steht,

$R^1$ und $R^2$,

die gleich oder verschieden sein können, für eine geradkettige oder verzweigte Alkylgruppe mit 1 bis 10 Kohlenstoffatomen, eine Alkenyl- oder Alkinylgruppe mit 2 bis 10 Kohlenstoffatomen, eine Aralkylgruppe mit 7 bis 16 Kohlenstoffatomen oder Wasserstoff stehen,

$R^3$ und $R^4$,

die gleich oder verschieden sein können, für eine geradkettige oder verzweigte Alkylgruppe mit 1 bis 4 Kohlenstoffatomen, eine Alkenyl- oder Alkinylgruppe mit 2 bis 10 Kohlenstoffatomen, eine geradkettige oder verzweigte Alkoxygruppe mit 1 bis 4 Kohlenstoffatomen, Wasserstoff, Fluor, Chlor oder Brom stehen oder für den Rest der Formel Ib stehen,

-COOR$^7$ Ib

wobei
$R^7$
eine geradkettige oder verzweigte Alkylgruppe mit 1 bis 4 Kohlenstoffatomen bedeutet und
X, Y und Z
für Sauerstoff steht,
ist bevorzugt.

Die Herstellung der Verbindung der Formel I erfolgt durch Umsetzung der Verbindung der Formel II,

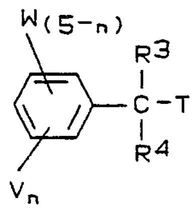

II

in der
V
für eine geradkettige oder verzweigte Alkylgruppe mit 1 bis 4 Kohlenstoffatomen, Fluor, Chlor, Brom, Jod oder Wasserstoff steht,
n
für eine ganze Zahl Von 1 bis 5 steht,
W
für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine geradkettige oder verzweigte Alkoxygruppe mit 1 bis 10 Kohlenstoffatomen, eine Alkylgruppe mit 1 bis 4 Kohlenstoffatomen, ein oder mehrfach substituiert durch den Rest der Formel

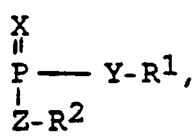

den Rest der Formel -COOR$^6$ oder Halogen, in der Halogen Chlor, Brom, Jod oder Fluor bedeutet, einen ankondensierten Aromaten, der durch eine Alkylgruppe mit 1 bis 4 Kohlenstoffatomen substituiert sein kann, Cyanid, Nitro oder Wasserstoff steht oder für den Rest der Formel Ia steht,

-COOR$^6$ Ia

wobei
$R^6$
eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff oder eine geradkettige oder verzweigte Halogenalkylgruppe mit 1 bis 20 Kohlenstoffatomen bedeutet, in der Halogen Chlor, Brom, Jod oder Fluor bedeutet,
T
für Chlor, Brom, Jod, Methylsulfonyl, Phenylsulfonyl oder Tosylsulfonyl steht,
$R^3$ und $R^4$,
die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Aralkylgruppe mit 7 bis 20 Kohlenstoffatomen, eine geradkettige oder verzweigte Alkoxygruppe mit 1 bis 10 Kohlenstoffatomen, Wasserstoff,

Fluor, Chlor, Brom oder Jod stehen, oder für den Rest der Formel Ib stehen,

-COOR$^7$ Ib

wobei
$R^7$
eine geradkettige oder verzweigte Alkylgruppe mit 1 bis 4 Kohlenstoffatomen bedeutet, mit der Verbindung der Formel III,

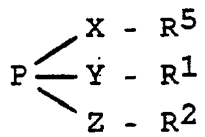

III

in der
$R^1$ und $R^2$,
die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff, eine geradkettige oder verzweigte Halogenalkylgruppe mit 1 bis 20 Kohlenstoffatomen stehen, in der Halogen Chlor, Brom, Jod oder Fluor bedeutet, oder $R^1$ und $R^2$ zusammen einen cyclischen Diester mit 2 bis 6 Kohlenstoffatomen im Ring bilden,
$R^5$
für eine geradkettige oder verzweigte Alkylgruppe mit 1 bis 4 Kohlenstoffatomen steht und
X, Y und Z,
die gleich oder verschieden sein können, für Sauerstoff oder Schwefel stehen.

Die Synthese der Verbindung der Formel I erfolgt durch Umsetzung der Verbindung der Formel II mit der Verbindung der Formel III, zweckmäßig bei Temperaturen zwischen 100 und 250 °C analog zu beschriebenen Methoden (US 4 299 615; Houben-Weyl, Methoden der Org. Chemie, Vol XII/1, Seite 423, Thieme Verlag Stuttgart; Houben-Weyl, Methoden der Org. Chemie, Vol E2, Seite 300). Die Reaktion kann in in einem geeigneten Lösungsmittel, wie Hexamethylphosphorsäureamid (HMPA), Dimethylformamid (DMF), Dimethylsulfoxid (DMSO), N,N'-Dimethyl-N,N'-propylenharnstoff (DMPU) oder N,N'-Dimethyl-N,N'-ethylenharnstoff (DMEU) erfolgen. Die Reaktion kann auch ohne Lösungsmittel durchgeführt werden. Die Reinigung erfolgt nach allgemein üblichen Methoden, bevorzugt durch Chromatographie über Kieselgel mit geeigneten Laufmitteln, durch Destillation oder durch Umkristallisation aus geeigneten Lösungsmitteln.

Verbindungen der Formel I können ferner aus anderen Verbindungen der Formel I hergestellt werden durch allgemein bekannte chemische Modifikation, wie z.B. elektrophile aromatische Substitution oder Oxidation von Alkyl-Seitenketten.

Die Verbindungen der Formel II und Formel III lassen sich nach bekannten Methoden darstellen. Die Überführung der Phosphonsäurediester in ihre Monoester sowie in die entsprechenden freien Säuren oder deren Salze erfolgt beispielsweise durch Kochen mit verdünnter Salzsäure (Houben-Weyl, Methoden der Organischen Chemie, Vol. XII/1, 1963, Seite 423), oder durch Umsetzung mit Trimethylbromsilan (C.E. McKenna, J. Schmidhauser, J.C.S. Chem. Commun., 1979, Seite 739). Die Reinigung erfolgt durch Umkristallisation in geeigneten Lösungsmitteln oder durch chromatographische Methoden, bevorzugt durch Ionenaustauschchromatographie mit geeigneten Laufmitteln.

Durch Ionenaustauschchromatographie können auch die gewünschten Salzformen erhalten werden.

Die Verbindung der Formel I besitzt wertvolle pharmakologische Eigenschaften, insbesondere eine antivirale Wirkung und zwar gegen sowohl durch DNA- als auch durch RNA-Viren verursachte Krankheiten, insbesondere gegen Krankheiten, die hervorgerufen werden durch Herpes simplex Virus (HSV I), Myxoviren, Friend-Leukämie-Virus (FLV) oder Human Immunodeficiency Virus (HIV). Die erfindungsgemäßen Verbindungen eignen sich daher zur Bekämpfung verschiedener, durch Viren verursachter Krankheiten, wie der Erkrankung des Respirationstraktes, Erkrankungen der Haut, des Auges, des zentralen Nervensystems, AIDS und AIDS verwandter Zustände, wie des AIDS verwandten Komplexen (ARC), generalisierte Lymphadenopathie (PGL), AIDS-verwandten, neuralgischen Zuständen (wie Schwachsinn oder tropische Paraparese), Anti-HIV-Antikörper-positive Zustände, Kaposi- Sarkom oder thrombopenische Purpura.

Ein Wirksamkeitstest von Chemotherapeutika für HIV-Infektionen beim Menschen bereitet Schwierigkeiten, da noch kein Infektionsmodell bei Labortieren existiert. Für die Prüfung von Chemotherapeutika muß deshalb auf die Infektion mit anderen Retroviren zurückgegriffen werden. In diesem Fall wurde die Infektion der Maus mit dem Friend-Leukämie-Virus gewählt. Dazu wurden normale NMRI-Labormäuse (NMRI = Naval Medical Research Institute) durch intravenöse Injektion mit Friend-Leukämie-Virus enthaltendem Mäuseserum infiziert. Bei den unbehandelten Kontrolltieren entwickelte sich als Symptom der Infektion innerhalb von 2 Wochen eine deutliche Vergrößerung von Milz und Leber. Die Behandlung erfolgte über 10 Tage, beginnend 48 Stunden nach der Infektion. Am 14. Versuchstag wurden die Tiere getötet und geöffnet. Die Milz wurde entnommen und gewogen. Als Meßparameter der therapeutischen Wirksamkeit wurde das Milzgewicht der behandelten Tiere mit dem der unbehandelten Infektionskontrolle in Relation gesetzt. Bei nicht infizierten ausgewachsenen Labormäusen (20 - 24 g Körpergewicht) erreichte die Milz etwa 1 % des Körpergewichts oder weniger, wahrend bei infizierten Tieren die Milz am Ende des Versuchs etwa 10 % des Körpergewichts wog.

Die Verbindung der Formel I kann entweder allein oder mit physiologisch verträglichen Hilfs- oder Trägerstoffen vermischt als Arzneimittel angewandt werden. Sie kann zu diesem Zweck oral in einer Dosis von 1 bis 500 mg/kg Tag, vorzugsweise 5 bis 50 mg/kg Tag verabreicht werden. Die Applikation für parenterale, rectale oder topische Anwendung oder als Aerosol erfolgt in einer Menge von 0,5 bis 500 mg/kg Tag, bevorzugt mit 2 bis 100 mg/kg Tag. Die Verbindung der Formel I wird zweckmäßig in Dosierungseinheiten verabreicht, die mindestens die wirksame Menge der erfindungsgemäßen Verbindungen, bevorzugt 25 bis 6000 mg, besonders bevorzugt 100 bis 1000 mg enthalten. Diese Werte beziehen sich auf einen erwachsenen Menschen mit einem Gewicht von 75 kg. Diese Dosiereinheiten können auch mehrmals pro Tag verabreicht werden. Die Dosierung kann in schweren Fällen auch erhöht werden. In vielen Fällen genügen jedoch auch geringere Mengen. Zur Bekämpfung von Krankheiten, die durch RNA- oder DNA-Viren verursacht werden, eignen sich besonders

2-Methylbenzylphosphonsäuredimethylester,
3-Brombenzylphosphonsäurediethylester,
2-Brombenzylphosphonsäurediethylester,
4-Brombenzylphosphonsäurediethylester,
2-Methylbenzylphosphonsäurediethylester,
3-Methylbenzylphosphonsäurediethylester,
4-Methylbenzylphosphonsäurediethylester,
2-Fluorbenzylphosphonsäurediethylester,
2-Chlorbenzylphosphonsäurediethylester,
2,5-Dimethylbenzylphosphonsäurediethylester,
2-Methylbenzylphosphonsäurediisopropylester,
2-Methylbenzylphosphonsäuredi-n-butylester,
2-Methylbenzylphosphonsäuredi-(2-chlorethyl)-ester,
2-Methylbenzylphosphonsäuredi-triethylammoniumsalz,
4-Chlorbenzylphosphonsäurediethylester,
2-Jodbenzylphosphonsäurediethylester,
4-Fluorbenzylphosphonsäurediethylester,
4-Jodbenzylphosphonsäurediisopropylester,
2-Nitrobenzylphosphonsäurediethylester,
2-Phenylethylphosphonsäurediethylester,
2-Carboxyethylbenzylphosphonsäurediethylester,
3-Carboxymethylbenzylphosphonsäurediethylester,
2-(Ethylacetyl)benzylphosphonsäurediethylester,
4-Methoxybenzylphosphonsäurediethylester,
2-Methyl-4-Brombenzylphosphonsäurediethylester,
4-Trifluormethylbenzylphosphonsäurediethylester,
1,2-Phenylen-bis(methylphosphonsäurediethylester),
1,3-Phenylen-bis(methylphosphonsäurediethylester),
2-Carboxybenzylphosphonsäurediethylester,
Benzylphosphonsäure-2-carbonsäure,
2-Carboxyethylbenzylphosphonsäureditriethylammoniumsalz,
1-Methylnaphthylphosphonsäurediethylester,
2-Methylnaphthylphosphonsäurediethylester,
2-Cyanobenzylphosphonsäurediethylester,

4-Cyanobenzylphosphonsäurediethylester,
2-Methylbenzylphosphonsäure.

Die erfindungsgemäße Verbindung der Formel I kann auch in Kombination mit anderen Wirkstoffen, insbesondere Antivirusmitteln und Immunstimulatoren, wie Interferonen verabreicht werden. Die Erfindung umfaßt weiterhin die Verwendung der erfindungsgemäßen Verbindung der Formel I bei der Herstellung von Arzneimitteln, die zur Behandlung und Prophylaxe der vorstehend genannten Krankheiten eingesetzt werden. Ein weiterer Gegenstand der Erfindung sind Arzneimittel, die eine oder mehrere erfindungsgemäße Verbindungen der Formel I enthalten.

Die Arzneimittel werden nach an sich bekannten, dem Fachmann geläufigen Verfahren hergestellt. Als Arzneimittel wird die erfindungsgemäße Verbindung der Formel I entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 % und 75 %, beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Verbindung der Formel I kann oral, parenteral, intravenös oder rectal appliziert werden, wobei neben der oralen Applikation insbesondere die intranasale Applikation als Aerosol bevorzugt ist.

Für eine orale Anwendungsform wird die Verbindung der Formel I mit den dafür geeigneten Zusatzstoffen wie Trägerstoffen, Stabilisatoren oder inerten Verdünnungsmitteln vermischt und durch die üblichen Methoden in eine geeignete Darreichungsformen gebracht, wie Tabletten, Dragees, Steckkapseln, wäßrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation wird die Verbindung der Formel I mit den dafür geeigneten Substanzen wie Lösungsvermittler, Emulgatoren oder weiteren Hilfsstoffen in Lösung, Suspension oder Emulsion gebracht. Als Lösungsmittel kommen z. B. physiologische Kochsalzlösung, Alkohole, z. B. Ethanol, Propanol, Glycerin, Zuckerlösungen wie Glucose- oder Mannitlösungen oder eine Mischung von Lösungsmitteln in Frage.

Die nachfolgenden Beispiele dienen der weiteren Erläuterung der Erfindung.

**Beispiel 1**

Herstellung von 2-Methylbenzylphosphonsäuredimethylester (A)

34,6 g (0,19 mol) 2-Methylbenzylbromid und 23,2 g (0,19 mol) Trimethylphosphit wurden für 3 h auf 105 °C erhitzt. Das entstandene Methylbromid wurde in einer Kühlfalle bei -70 °C aufgefangen. Das Produkt wurde durch fraktionierte Destillation gereinigt.

Ausbeute: 22,5 g ( 57 %) Sdp.: 106 °C/0,4 mm; $^{1}H$-NMR (60 MHz, $CDCl_3$/TMS): $\delta$ = 2,33 (s,3H,Ar-$CH_3$), 3,22 (d,2H,$CH_2$-P) $J_{PH}$ = 22 Hz, 3,66 (d,6H,P-O-$\underline{CH_3}$), 7,26 (s-breit, 4H,Ar-H).

**Beispiel 2**

Herstellung von 3-Brombenzylphosphonsäurediethylester (B)

50 g (0,2 mol) 3-Brombenzylbromid und 33,2 g (0,2 mol) Triethylphosphit wurden 2 h auf 140 °C erhitzt, dabei destillierte Ethylbromid ab. Das Produkt wurde über eine 30 cm Vigreux-Kolonne destilliert.

Ausbeute: 44,02 g (72 %); Sdp.: 140-148 °C/0,7-0,8 mm; $^{1}H$-NMR (60 MHz, $CDCl_3$/TMS): $\delta$ = 1,21 (t,6H,P-O-$CH_2$-$\underline{CH_3}$), 3,10 (d,2H,$CH_2$-P) $J_{PH}$ = 22 Hz,4,03 (dq,4H,P-O-$\underline{CH_2}$-$CH_3$), 7,04-7,61 (m,4H,Ar-H).

**Beispiel 3**

Herstellung von 2-Brombenzylphosphonsäurediethylester (C)

Verfahren wie in Beispiel 2. Es wurde 2-Brombenzylbromid statt 3-Brombenzylbromid verwendet.

Ausbeute: 78 % Sdp.: 120 °C/0,2 mm; $^{1}H$-NMR (60 MHz, $CDCl_3$/TMS): $\delta$ = 1,25 (t,6H,P-O-$CH_2$-$\underline{CH_3}$), 3,4 (d,2H,$CH_2$-P) $J_{PH}$ = 22 Hz, 4,06 (dq,4H,P-O-$\underline{CH_2}$-$CH_3$), 7,06-7,70 (m,4H,Ar-H)

**Beispiel 4**

Herstellung von 4-Brombenzylphosphonsäurediethylester (D)
Verfahren wie in Beispiel 2. Es wurde 4-Brombenzylbromid statt 3-Brombenzylbromid verwendet.
Ausbeute: 66 %; Sdp.: 135° C/0,3 mm; $^1$H-NMR (60 MHz, $CDCl_3$/TMS): δ 1,23 (t,6H,P-O-$CH_2$-$\underline{CH_3}$), 3,10 (d,2H,$CH_2$-P) $J_{PH}$ = 22 Hz, 4,03 (dq,4H,P-O-$\underline{CH_2}$-$CH_3$), 6,99-7,62 (m,4H,Ar-H)

**Beispiel 5**

Herstellung von 2-Methylbenzylphosphonsäurediethylester (E)
Verfahren wie in Beispiel 2. Es wurde 2-Methylbenzylbromid statt 3-Brombenzylbromid verwendet.
Ausbeute: 75 %; Sdp.: 122° C C/4 mm; $^1$H-NMR (270 MHz, $CDCl_3$/TMS): δ = 1,25 (t,6H,P-O-$CH_2$-$\underline{CH_3}$), 2,39 (s,3H,Ar-$CH_3$), 3,18 (d,2H,$CH_2$-P) $J_{PH}$ = 24 Hz, 4,00 (dq,4H,P-O-$\underline{CH_2}$-$CH_3$), 7,10-7,31 (m,4H,Ar-H).

**Beispiel 6**

Herstellung von 3-Methylbenzylphosphonsäurediethylester (F)
Verfahren wie in Beispiel 2. Es wurde 3-Methylbenzylbromid statt 3-Brombenzylbromid verwendet.
Ausbeute: 84 %; Sdp.: 108° C/0,2 mm; $^1$H-NMR (60 MHz, $CDCl_3$/TMS): δ = 1,21 (t,6H,P-O-$CH_2$-$\underline{CH_3}$), 2,30 (s,3H,Ar-$CH_3$), 3,13 (d,2H,$CH_2$-P) $J_{PH}$ = 22 Hz, 4,03 (dq,4H,P-O-$\underline{CH_2}$-$CH_3$), 7,13 (s-breit,4H,Ar-H).

**Beispiel 7**

Herstellung von 4-Methylbenzylphosphonsäurediethylester (G)
Verfahren wie in Beispiel 2. Es wurde 4-Methylbenzylbromid statt 3-Brombenzylbromid verwendet.
Ausbeute: 83 %; Sdp.: 110-115° C/0,15 mm; $^1$H-NMR (60 MHz, $CDCl_3$/TMS): δ = 1,22 (t,6H,P-O-$CH_2$-$\underline{CH_3}$), 2,35 (s,3H,Ar-$CH_3$), 3,10 (d,2H,$CH_2$-P) $J_{PH}$ = 22 Hz, 4,03 (dq,4H,P-O-$\underline{CH_2}$-$CH_3$), 7,15 (s,4H,Ar-H).

**Beispiel 8**

Herstellung von 2-Fluorbenzylphosphonsäurediethylester (H)
Verfahren wie in Beispiel 2. Es wurde 2-Fluorbenzylbromid statt 3-Brombenzylbromid verwendet.
Ausbeute: 83 %; Sdp.: 105-114° C/0,6 mm; $^1$H-NMR (60 MHz, $CDCl_3$/TMS): δ = 1,23 (t,6H,P-O-$CH_2$-$\underline{CH_3}$), 3,20 (d,2H,$CH_2$-P) $J_{PH}$ = 22 Hz, 4,02 (dq,4H,P-O-$\underline{CH_2}$-$CH_3$), 6,85-7,70 (m,4H,Ar-H).

**Beispiel 9**

Herstellung von 2-Chlorbenzylphosphonsäurediethylester (I)
Verfahren wie in Beispiel 2. Es wurde 2-Chlorbenzylbromid statt 3-Brombenzylbromid verwendet.
Ausbeute: 68 %; Sdp.: 140-145° C/0,002 mm; $^1$H-NMR (60 MHz, $CDCl_3$/TMS): δ = 1,20 (t,6H, P-O-$CH_2$-$\underline{CH_3}$), 3,38 (d,2H,$CH_2$-P) $J_{PH}$= 22 Hz, 4,02 ($d_q$,4H, P-O-$\underline{CH_2}$-$CH_3$), 7,00-7,65 (m,4H,Ar-H)

**Beispiel 10**

Herstellung von 2,5-Dimethylbenzylphosphonsäurediethylester (K)
Verfahren wie in Beispiel 2. Es wurde 2,5-Dimethylbenzylbromid statt 3-Brombenzylbromid verwendet.
Ausbeute: 80 %; Sdp.: 116° C/0,2 mm; $^1$H-NMR (60 MHz, $CDCl_3$/TMS): δ = 1,29 (t,6H,P-O-$CH_2$-$\underline{CH_3}$), 2,34 (s,6H,Ar-$CH_3$), 3,20 (d,2H,$CH_2$-P) $J_{PH}$= 22 Hz, 4,05 (dq,4H,P-O-$\underline{CH_2}$-$CH_3$), 7,07 (s-breit, 4H, Ar-H)

**Beispiel 11**

Herstellung von 2-Methylbenzylphosphonsäurediisopropylester (L)
34,5 g (0,19 mol) 2-Methylbenzylbromid und 39 g (0,19 mol) Triisopropylphosphit wurden 2 h auf 150° C erhitzt. Dabei destillierte i-Propylbromid ab. Das Produkt wurde über eine 30 cm Vigreux-Kolonne destilliert.
Ausbeute: 78 %; Sdp.: 110° C/0,35 mm; $^1$H-NMR (60 MHz, $CDCl_3$/TMS): δ = 1,26 (t,6H,P-O-CH($\underline{CH_3}$)$_2$), 2,37 (s,3H,Ar-$CH_3$), 3,13 (d,2H,$CH_2$-P) $J_{PH}$= 22 Hz, 4,56 (dhep,2H,P-O-CH($\underline{CH_3}$)$_2$), 7,15 (s-breit, 4H, $\underline{Ar\text{-}H}$).

**Beispiel 12**

Herstellung von 2-Methylbenzylphosphonsäuredi-n-butylester (M)

34,5 g (0,19 mol) 2-Methylbenzylbromid und 46,8 g (0,19 mol) Tri-n-butylphosphit wurden 2 h auf 160° C erhitzt. Dabei destillierte n-Butylbromid ab. Das Produkt wurde wie in Beispiel 11 destilliert.

Ausbeute: 55 %; Sdp.: 143° C/0,5 mm; $^1$H-NMR (60 MHz, $CDCl_3$/TMS): δ = 0,64-1,77 (m,14H,$\underline{CH_2}$-$\underline{CH_2}$-$\underline{CH_3}$), 2,39 (s,3H,Ar-$CH_3$), 3,18 (d,2H,$CH_2$-P) $J_{PH}$ = 22 Hz, 3,95 (dt,4H,P-O-$\underline{CH_2}$-), 7,20 (s-breit, 4H, $\underline{\text{Ar-H}}$)

**Beispiel 13**

Herstellung von 2-Methylbenzylphosphonsäuredi-(2-chlorethyl)ester (N)

34,5 g (0,19 mol) 2-Methylbenzylbromid und 50,4 g (0,19 mol) Tri-(2-chlorethyl))phosphit wurden 2 h auf 160° C erhitzt.

Dabei destillierte 1-Brom-2-chlorethan ab. Das Produkt wurde wie in Beispiel 11 destilliert.

Ausbeute: 82 %; Sdp.: 170° C/0,5 mm; $^1$H-NMR (60 MHz, $CDCl_3$/TMS): δ = 2,41 (s,3H,Ar-$CH_3$), 3,29 (d,2H,$CH_2$-P) $J_{PH}$ = 22 Hz, 3,59 (m,4H,O-$CH_2$-$\underline{CH_2}$-Cl) 4,18 (m,4H,O-$\underline{CH_2}$-$CH_2$-Cl), 7,11-7,33 (m,4H,Ar-H)

**Beispiel 14**

Herstellung von 2-Methylbenzylphosphonsäure-ditriethylammoniumsalz (O)

Zu 2 g (9,3 mmol) der Verbindung (E) in 10 ml absoluten Dioxan wurden 3,2 g (21 mmol) Trimethylsilylbromid getropft, das Reaktionsgemisch wurde auf 50° C erhitzt und 6 h bei dieser Temperatur gerührt. Es wurde evaporiert, mehrfach mit Wasser versetzt und lyophylisiert. Das Rohprodukt wurde durch Chromatographie über DEAE ®Sephadex A25 ($Et_3NH^+$ Form, Firma Pharmacia, Freiburg, BRD) und Elution mit einem Triethylammoniumbicarbonat Gradienten von 0,3-1,0 M gereinigt.

Ausbeute: 1,21 g (61 %); Öl; $^1$H-NMR (60 MHz, DMSO/TMS): δ = 1,28 (t,18H,N-$CH_2$-$\underline{CH_3}$), 2,35 (s,3H,Ar-$CH_3$), 3,10 (d,2H,$CH_2$-P) $J_{PH}$ = 22 Hz, 3,16 (q,12H,N-$\underline{CH_2}CH_3$), 7,26 (s-breit,4H,Ar-H).

**Beispiel 15**

Herstellung von 4-Chlorbenzylphosphonsäurediethylester (P)

Verfahren wie in Beispiel 2. Es wurde 4-Chlorbenzylbromid statt 3-Brombenzylbromid verwendet. Ausbeute: 78 %; Sdp.: 123° C/0,4 mm; $^1$H-NMR (60 MHz, $CDCl_3$/TMS): δ = 1,25 (t, 6H, P-O-$CH_2$-$\underline{CH_3}$); 3,10 (d,2H,$CH_2$-P) $J_{PH}$ = 22 Hz, 4,05 (dq,4H, P-O-$\underline{CH_2}$-$CH_3$), 7,30 (s,4H,Ar-H)

**Beispiel 16**

Herstellung von 2-Jodbenzylphosphonsäurediethylester (R)

Verfahren wie in Beispiel 2. Es wurde 2-Jodbenzylbromid statt 3-Brombenzylbromid verwendet. Ausbeute: 69 %; Sdp.: 113° C/0,1 mm; $^1$H-NMR (60 MHz, $CDCl_3$/TMS): δ = 1,30 (t,6H, P-O-$CH_2$-$\underline{CH_3}$), 3,57 (d,2H,$CH_2$-P) $J_{PH}$ = 21 Hz, 4,10 (dq,4H, P-O-$\underline{CH_2}$-$CH_3$), 6,80-8,03 (m,4H,Ar-H).

**Beispiel 17**

Herstellung von 4-Fluorbenzylphosphonsäurediethylester (S)

Verfahren wie in Beispiel 2. Es wurde 4-Fluorbenzylbromid statt 3- Brombenzylbromid verwendet. Ausbeute: 84 %; Sdp.: 90° C/0,25 mm; $^1$H-NMR (60 MHz, $CDCl_3$/TMS): δ = 1,30 (t,6H, P-O-$CH_2$-$\underline{CH_3}$), 3,13 (d,2H,$CH_2$-P) $J_{PH}$ = 22 Hz, 4,07 (dq,4H, P-O-$\underline{CH_2}$-$CH_3$), 6,85-7,53 (m,4H,Ar-H).

**Beispiel 18**

Herstellung von 4-Jodbenzylphosphonsäurediisopropylester (T)

Verfahren wie in Beispiel 11. Es wurde 4-Jodbenzylbromid statt 2-Methylbenzylbromid verwendet. Ausbeute: 78 %; Sdp.: 137° C/0,1 mm; $^1$H-NMR (60 MHz, $CDCl_3$/TMS): δ = 1,20 (t,6H, P-O-CH($\underline{CH_3}$)$_2$), 3,05 (d,2H,$CH_2$-P) $J_{PH}$ = 22 Hz, 4,60 (dhep,2H, P-O-CH($\underline{CH_3}$)$_2$), 6,93-7,80 (m,4H,Ar-H).

**Beispiel 19**

2-Nitrobenzylphosphonsäurediethylester (U)

Verfahren wie in Beispiel 2; Verwendung von 2-Nitrobenzylbromid anstelle von 3-Brombenzylbromid. Ausbeute: 73 %; Sdp.: 158° C/0,4 Torr; $^1$H-NMR (60 MHZ, $CDCl_3$/TMS): δ = 1,20 (t,6H,P-O-$CH_2$-$CH_3$); 3,70 (d,2H,P-$CH_2$, $J_{PH}$ = 23 Hz); 4,01 (dq, 4H,P-O-$CH_2$-$CH_3$); 7,06-8,10 (m,4H, Ar-H).

**Beispiel 20**

2-Phenylethylphosphonsäurediethylester (V)

Verfahren wie in Beispiel 2; Verwendung von 1-Phenylethylbromid anstelle von 3-Bromhenzylbromid. Ausbeute: 60 %; Sdp.: 122° C/0,05 Torr; $^1$H-NMR (270 MHz, $CDCl_3$/TMS): δ = 1,15 & 1,29 (t,6H,P-O-$CH_2$-$CH_3$); 1,55 & 1,62 (d,3H,CH-$CH_3$); 3,18 (dq,1H,P-CH, $J_{PH}$ = 23 Hz); 3,71-4,20 (m,4H,P-O-$CH_2$-$CH_3$); 7,20-7,46 (m,5H,Ar-H).

**Beispiel 21**

2-Carboxyethylbenzylphosphonsäurediethylester (W)

82,1 g (0,5 mol) o-Tolylsäuremethylester wurden in 375 ml $CCl_4$ gelöst und 94 g (0,5 mol) N-Bromsuccinimid (NBS) und 0,3 g Azobisisobutyronitril (AIBN) zugegeben. Unter Rühren wurde 3 h am Rückfluß gekocht, abgekühlt, entstandenes Succinimid wurde abfiltriert und mit $CCl_4$ nachgewaschen. Das Lösungsmittel wurde einrotiert. Der entstandene o-Brommethylbenzoesäureethylester (131,7 g) wurde ohne weitere Reinigung wie unter Beispiel 2 beschrieben weiter umgesetzt.

Ausbeute: 63 %; Sdp.: 130° C/0,2 Torr; $^1$H-NMR (270 MHz,$CDCl_3$/ TMS): δ = 1,22 (t,6H,P-O-$CH_2$-$CH_3$); 1,39 (t,3H,C-O-$CH_2$-$CH_3$); 3,41 (d,2H,P-$CH_2$, $J_{PH}$ = 23 Hz); 3,99 (dq,4H,P-O-$CH_2$-$CH_3$); 4,38 (q,2H,C-O-$CH_2$-$CH_3$); 7,25-7,49 (m,3H,Ar-H); 7,92 (d,1H, Ar-H).

**Beispiel 22**

3-Carboxymethylbenzylphosphonsäurediethylester (X)

m-Tolylsäuremethylester wurde wie in Beispiel 21 beschrieben mit NBS und AIBN zu m-Brommethylbenzoesäuremethylester bromiert und dieser ohne weitere Reinigung mit Triethylphosphit wie in Beispiel 2 beschrieben weiter umgesetzt.

Ausbeute: 45 %; Sdp.: 146° C/0,5 Torr; $^1$H-NMR (60 MHz, $CDCl_3$/ TMS): δ = 1,25 (t,6H,P-O-$CH_2$-$CH_3$); 3,21 (d,2H,P-$CH_2$, JPH = 22 Hz); 3,93 (s,3H,$OCH_3$); 4,02 (dq,4H,P-O-$CH_2$-$CH_3$); 7,25-8,10 (m,4H,Ar-H).

**Beispiel 23**

2-(Ethylacetyl)benzylphosphonsäurediethylester (Y)

o- Tolylessigsäure wurde wie in Beispiel 21 beschrieben mit NBS und AIBN zu o-Brommethylphenylessigsäureethylester bromiert und dieser wie in Beispiel 2 beschrieben mit Triethylphosphit umgesetzt.

Ausbeute: 33 %; Sdp.: 152° C/0,3 Torr; $^1$H-NMR (60 MHz,$CDCl_3$/ TMS): δ = 1,23 (t,9H,P-O-$CH_2$-$CH_3$ & C-O-$CH_2$-$CH_3$); 3,29 (d,2H,P-$CH_2$, $J_{PH}$ = 22 Hz); 3,70-4,28 (m,8H,P-O-$CH_2$-$CH_3$ & C-O-$CH_2$-$CH_3$ & Ar-$CH_2$-C(O)); 7,30 (s,4H,Ar-H).

**Beispiel 24**

4-Methoxybenzylphosphonsäurediethylester (Z)

p-Methoxybenzylchlorid wurden wie in Beispiel 2 beschrieben mit Triethylphosphit umgesetzt. Ausbeute: 88 %; Sdp.: 127° C/0,2 Torr; $^1$H-NMR (270 MHz,$CDCl_3$/ TMS): δ = 1,25 (t,6H,P-O-$CH_2$-$CH_3$); 3,11 (d,2H,P-$CH_2$, $J_{PH}$ = 23 Hz); 3,80 (s,3H,$OCH_3$); 4,07 (dq,4H,P-O-$CH_2$-$CH_3$); 6,73-7,32 (m,4H,Ar-H).

**Beispiel 25**

2-Methyl-4-Brombenzylphosphonsäurediethylester (AA)

10 g (42 mmol) 2-Methylbenzylphosphonsäurediethylester wurden in 60 ml Trimethylphosphat gelöst und in einem gegen Licht und Feuchtigkeit geschützten Kolben eingebracht. Unter Rühren wurden 16 g (0,05 mol) $Br_2$ zugetropft. Es wurde 15 h bei 90° C gerührt, nach dem Abkühlen mit 100 ml Wasser verdünnt und dreimal mit je 100 ml n-Hexan extrahiert. Die org. Phase wurde getrocknet, eingeengt und destilliert.

Ausbeute: 35 %; Sdp.: 124°C/0,2 mm; $^1$H-NMR (60 MHz,$CDCl_3$/ TMS): δ = 1,25 (t,6H,P-O-$CH_2$-$\underline{CH_3}$); 2,30 (s,3H,Ar-$CH_3$); 3,17 (d,2H,P-$CH_2$, $J_{PH}$ = 23 Hz); 4,06 (dq,4H,P-O-$\underline{CH_2}$-$CH_3$); 6,93-7,88 (m,3H,Ar-H).

**Beispiel 26**

4-Trifluormethylbenzylphosphonsäurediethylester (AB)

10 g (0,056 mol) 4-Trifluormethylbenzylalkohol wurden mit 13,5 g (0,114 mol) $SOCl_2$ gemischt und fünf Stunden unter Rückfluß gekocht. Überschüssiges $SOCl_2$ wurde abdestilliert. Das entstandene 4-Trifluormethylbenzylchlorid wurde ohne weitere Reinigung mit Triethylphosphit, wie in Beispiel 2 beschrieben, umgesetzt.

Ausbeute: 48 %; Sdp.: 90°C/0,45 Torr; $^1$H-NMR (60 MHz,$CDCl_3$/ TMS): δ = 1,27 (t,6H,P-O-$CH_2$-$\underline{CH_3}$); 3,21 (d,2H,P-$CH_2$, $J_{PH}$ = 23 Hz); 4,10 (dq,4H,P-O-$\underline{CH_2}$-$CH_3$); 7,27-7,77 (m,4H,Ar-H).

**Beispiel 27**

1,2-Phenylen-bis(methylphosphonsäurediethylester) (AG)

1,2-Dibrommethylbenzol wurde mit zwei Äquivalenten Triethylphosphit wie in Beispiel 2 beschrieben umgesetzt.

Ausbeute: 68 %; Sdp.: 161°C/0,15 Torr; $^1$H-NMR (270 MHz,$CDCl_3$/ TMS): δ = 1,25 (t,12H,P-O-$CH_2$-$\underline{CH_3}$); 3,43 (d,4H,P-$CH_2$, $J_{PH}$ = 21 Hz); 4,00 (dq,8H,P-O-$\underline{CH_2}$-$CH_3$); 7,16-7,32 (m,4H,Ar-H).

**Beispiel 28**

1,3-Phenylen-bis(methylphosphonsäurediethylester (AD)

1,3-Dibrommethylbenzol wurde mit zwei Äquivalenten Triethylphosphit wie in Beispiel 2 beschrieben umgesetzt.

Ausbeute: 63 %; Sdp.: 165-169°C/0,1 Torr; $^1$H-NMR (60 MHz,$CDCl_3$/TMS): δ = 1,27 (t,12H,P-O-$CH_2$-$\underline{CH_3}$); 3,16 (d,4H,P-$CH_2$, $J_{PH}$ = 24 Hz); 4,07 (dq,8H,P-O-$\underline{CH_2}$-$CH_3$); 7,25 (s,4H,Ar-H).

**Beispiel 29**

2-Carboxybenzylphosphonsäurediethylester (AE)

Zu einer Lösung von 12 g $KMnO_4$ und 4 g $Na_2CO_3$ in 300 ml Wasser wurden 4 g 2-Methylbenzylphosphonsäurediethylester zugegeben und 24 h am Rückfluß gekocht. Es wurde mit 15 ml konz. $H_2SO_4$ angesäuert und fünf mal mit je 300 ml Ether extrahiert. Die org. Phase wurde über $MgSO_4$ getrocknet, das Lösungsmittel einrotiert, der Rückstand über Kieselgel ($CH_2Cl_2$/Methanol/Triethylamin 9/1/0,1) chromatographiert. Ausbeute: 20 %; $^1$H-NMR (270 MHz,DMSO/TMS): δ = 1,13 (t,6H,P-O-$CH_2$-$\underline{CH_3}$); 3,29 (d,2H,P-$CH_2$, $J_{PH}$ = 23 Hz); 3,90 (dq,4H,P-O-$\underline{CH_2}$-$CH_3$); 7,38-7,88 (m,4H,Ar-H).

**Beispiel 30**

Benzylphosphonsäure-2-carbonsäure (AF)

5 g 2-Carboxyethylbenzylphosphonsäurediethylester wurden mit 100 ml 3/4 konz. HCl versetzt und 5 h unter Rückfluß erhitzt. Beim Abkühlen kristallisierte das Produkt aus.

Ausbeute: 50 %; Fp.: 209°C; $^1$H-NMR (60 MHz,$D_2O$/TMS): δ = 3,66 (d,2H,P-$CH_2$, $J_{PH}$ = 23 Hz); 7,21-8,07 (m,4H,Ar-H).

**Beispiel 31**

2-Carboxyethylbenzylphosphonsäureditriethylammoniumsalz (AG)

Verfahren wie in Beispiel 14. Es wurde 2-Carboxyethylbenzylphosphonsäurediethylester anstelle von 2-Methylbenzylphosphonsäurediethylester verwendet.

Ausbeute: 60 %; Harz; $^1$H-NMR (270 MHz,$D_2O$/TMS): δ = 1,28 (t,18H,N-$CH_2$-$\underline{CH_3}$); 1,39 (t,3H,O-$CH_2$-$\underline{CH_3}$); 3,19 (q,12H,N-$\underline{CH_2}$-$CH_3$); 3,50 (d,2H,P-$CH_2$, $J_{PH}$ = 21 Hz); 4,49 (q,2H,O-$\underline{CH_2}$-$CH_3$); 7,31-7,83 (m,4H,Ar-H).

**Beispiel 32**

1-Methylnaphthylphosphonsäurediethylester (AN)

Verfahren wie in Beispiel 2; Verwendung von 1-Chlormethylnaphthalin anstelle von 3-Brombenzylbromid. Ausbeute: 71 %; Sdp.: 150-155° C/0,15 Torr; $^1$H-NMR (270 MHz, $CDCl_3$/TMS): δ = 1,15 (t,6H,P-O-$CH_2$-$\underline{CH_3}$); 3,65 (d,2H,P-$CH_2$, $J_{PH}$ = 23 Hz); 3,94 (dq,4H,P-O-$\underline{CH_2}$-$CH_3$); 7,39-8,15 (m,7H,Ar-H).

**Beispiel 33**

2-Methylnaphthylphosphonsäurediethylester (AH)

Verfahren wie in Beispiel 2; Verwendung von 2-Brommethylnaphthalin anstelle von 3-Brombenzylbromid.

Ausbeute: 67 %; Sdp.: 165-170° C/0,15 Torr; $^1$H-NMR (60 MHz, $CDCl_3$/TMS): δ = 1,23 (t,6H,P-O-$CH_2$-$\underline{CH_3}$); 3,32 (d,2H,P-$CH_2$, $J_{PH}$ = 22 Hz); 4,04 (dq,4H,P-O-$\underline{CH_2}$-$CH_3$); 7,28-8,07 (m,7H,Ar-H).

**Beispiel 34**

2-Cyanobenzylphosphonsäurediethylester (AK)

Verfahren wie in Beispiel 2; Verwendung von 2-Brommethylbenzonitril anstelle von 3-Brombenzylbromid.

Ausbeute: 44 %; Sdp.: 154° C/0,1 Torr; $^1$H-NMR (60 MHz,$CDCl_3$/ TMS): δ = 1,30 (t,6H,P-O-$CH_2$-$\underline{CH_3}$); 3,41 (d,2H,P-$\underline{CH_2}$, $J_{PH}$ = 22 Hz); 4,13 (dq,4H,P-O-$\underline{CH_2}$-$CH_3$); 7,17-7,86 (m,4H,Ar-H).

**Beispiel 35**

4-Cyanobenzylphosphonsäurediethylester (AL)

Verfahren wie in Beispiel 2; Verwendung von 4-Brommethylbenzonitril anstelle von 3-Brombenzylbromid. Ausbeute: 44 %; Sdp.: 154° C/0,1 Torr; $^1$H-NMR (60 MHz,$CDCl_3$/ TMS): δ = 1,25 (t,6H,P-O-$CH_2$-$CH_3$); 3,20 (d,2H,P-$CH_2$, $J_{PH}$ = 22 Hz); 4,05 (dq,4H,P-O-$\underline{CH_2}$-$CH_3$); 6,98-7,71 (m,4H,Ar-H).

**Beispiel 36**

2-Methylbenzylphosphonsäure (AM)

Verfahren wie in Beispiel 28. Verwendung von 2-Methylbenzylphosphonsäurediethylester anstelle von 2-Carboxyethylbenzylphosphonsäurediethylester.

Ausbeute: 77 %; Fp.: 199° C; $^1$H-NMR (270 MHz,DMSO/TMS): δ = 2,32 (s,3H,$CH_3$); 2,95 (d,2H,P-$CH_2$, JPH = 22 Hz); 7,03-7,22 (m,4H,Ar-H).

**Beispiel 37**

Pathogenfreie NMRI-Mäuse (NMRI:Naval Medical Research Institute) mit einem Gewicht von etwa 15 g wurden intraperitoneal mit Herpes simplex Typ 1 infiziert und anschließend mit den in Tabelle 1 genannten Verbindungen intraperitoneal, subkutan oder oral therapiert. Die Behandlung erfolgte zweimal täglich, beginnend nach der Infektion. Die Substanzen wurden den Versuchstieren 5 bzw. 9 mal (siehe Tabelle 1) verabreicht. Der Behandlungserfolg wurde anhand des Krankheitsverlaufes und der Überlebensrate gegenüber den unbehandelten Kontrolltieren bestimmt. Diese Tiere erhielten anstelle der zu prüfenden Verbindung eine wasserlösliche Methylhydroxyethylcellulose (Viskosität 300 Pa•s in 2 % Lösung) appliziert. Die Versuche wurden mit Gruppen von je 5 Mäusen pro Substanz durchgeführt.

Die chemotherapeutische Wirkung wird aus Tabelle 1 ersichtlich.

**Beispiel 38**

Zellkulturen von Hela- und Vero-Zellen wurden in Mikrotiterplatten überimpft und mit Myxoviren (Influenza A2) infiziert. 2 Stunden nach der Infektion wurde die Verbindung (O) den infizierten Zellkulturen in verschiedenen Verdünnungen zugegeben. 49 bis 72 Stunden nach der Infektion wurde der Therapieerfolg anhand des cytopathogenen Effektes mikroskopisch und nach Neutralrotaufnahmen (Farbtest nach Finter) photometrisch bestimmt (N. B. Finter, Interferons North Holland Publishing Co., Amsterdam, 1966). Die minimale Konzentration, bei der etwa die Hälfte der infizierten Zellen keinen cytopathogenen Effekt zeigen, wird als minimale Hemmkonzentration (MHK) betrachtet. Die Ergebnisse sind in Tabelle 2 zusammengefaßt.

**Tabelle 2**

| | Influenza A2 | |
|---|---|---|
| Substanz | MHK (µg/ml) | DTM (µg/ml) |
| 0 | 14,8 | 133,3 |

DTM = Dosis tolerata maxima

**Beispiel 39**

Pathogenfreie NMRI-Mäuse mit einem Gewicht von etwa 16 g wurden intranasal mit Influenza A2 infiziert und anschließend mit den in Tabelle 3 genannten Verbindungen subkutan oder oral therapiert. Als Vergleich diente stets Amantadin. Die Behandlung erfolgte zweimal täglich über 2,5 Tage, beginnend nach der Infektion. Der Behandlungserfolg wurde anhand des Krankheitsverlaufes und der Überlebensrate gegenüber den unbehandelten Kontrolltieren bestimmt. Diese Tiere erhielten anstelle der zu prüfenden Substanz eine wasserlösliche

**Tabelle 1**

Herpes simplex 1

| Substanz | | Dosierung (mg/kg) | Überlebende | Mittlere Überlebenszeit (Tage) |
|---|---|---|---|---|
| A | po | 5 x 2,5 | 3 | 10,5 |
| A | po | 5 x 25 | 3 | 9,0 |
| Kontrolle | po | 5 x 0 | 2 | 9,7 |
| | | | | |
| A | sc | 5 x 2,5 | 2 | 12,0 |
| A | sc | 5 x 25 | 4 | 9,0 |
| A | sc | 5 x 250 | 4 | 9,0 |
| Kontrolle | sc | 5 x 0 | 1 | 7,5 |
| | | | | |
| C | po | 5 x 2,5 | 2 | 6,7 |
| C | po | 5 x 25 | 3 | 8,5 |
| C | po | 5 x 250 | 2 | 8,0 |
| Kontrolle | po | 5 x 0 | 2 | 8,0 |
| | | | | |
| D | po | 5 x 0,25 | 2 | 8,7 |
| D | po | 5 x 2,5 | 2 | 7,0 |
| D | po | 5 x 25 | 3 | 6,0 |
| Kontrolle | po | 5 x 0 | 0 | 8,0 |
| | | | | |
| D | po | 9 x 3,0 | 4 | 8,0 |
| D | po | 9 x 10,0 | 2 | 8,3 |
| D | po | 9 x 30,0 | 3 | 10,5 |
| Kontrolle | po | 9 x 0 | 1 | 7,5 |
| | | | | |
| D | sc | 5 x 0,25 | 1 | 9,0 |
| D | sc | 5 x 2,5 | 3 | 8,5 |
| D | sc | 5 x 25 | 4 | 8,0 |
| Kontrolle | sc | 5 x 0 | 1 | 7,7 |
| | | | | |
| E | po | 9 x 3 | 1 | 5,7 |
| E | po | 9 x 10 | 1 | 9,3 |
| E | po | 9 x 30 | 3 | 7,5 |
| Kontrolle | po | 9 x 0 | 2 | 7,0 |

**Fortsetzung Tabelle 1**

Herpes simplex 1

| Substanz | | Dosierung (mg/kg) | Überlebende | Mittlere Überlebenszeit (Tage) |
|---|---|---|---|---|
| E | sc | 9 x 3 | 1 | 6,3 |
| E | sc | 9 x 10 | 3 | 6,5 |
| E | sc | 9 x 30 | 2 | 6,7 |
| Kontrolle | sc | 9 x 0 | 1 | 8,3 |
| K | sc | 5 x 2,5 | 0 | 7,4 |
| K | sc | 5 x 25 | 0 | 8,6 |
| K | sc | 5 x 250 | 3 | 8,0 |
| Kontrolle | sc | 5 x 0 | 0 | 7,0 |
| L | po | 5 x 2,5 | 5 | - |
| L | po | 5 x 25 | 2 | 8,7 |
| L | po | 5 x 250 | 2 | 8,7 |
| Kontrolle | po | 5 x 0 | 2 | 9,7 |
| L | sc | 5 x 2,5 | 1 | 8,3 |
| L | sc | 5 x 25 | 0 | 7,4 |
| L | sc | 5 x 250 | 3 | 6,5 |
| Kontrolle | sc | 5 x 0 | 1 | 7,5 |
| M | sc | 5 x 2,5 | 4 | 7,0 |
| M | sc | 5 x 25 | 3 | 7,5 |
| M | sc | 5 x 250 | 1 | 7,7 |
| Kontrolle | sc | 5 x 0 | 1 | 7,5 |
| W | po | 9 x 3,0 | 1 | 8,5 |
| W | po | 9 x 10 | 1 | 9,5 |
| W | po | 9 x 30 | 4 | 8,0 |
| Kontrolle | po | 9 x 0 | 1 | 8,7 |

po = oral
sc = subkutan

Methylhydroxyethylcellulose (Viskosität 300 Pa•s in 2 % Lösung) appliziert. Die Versuche wurden mit Gruppen von je 5 Mäusen pro Substanz durchgeführt.

Die chemotherapeutische Wirkung wird in Tabelle 3 gezeigt.

Tabelle 3

Influenza A2

| Substanz | | Dosierung (mg/kg) | Überlebende | Mittlere Überlebenszeit (Tage) |
|---|---|---|---|---|
| Kontrolle | po | 0,0 | 2 | 7,7 |
| Amantadin | po | 80,0 | 5 | --- |
| A | po | 0,25 | 3 | 6,0 |
| A | po | 2,5 | 4 | 7,0 |
| A | po | 25,0 | 5 | --- |
| Kontrolle | sc | 0,0 | 1 | 6,5 |
| Amantadin | sc | 80,0 | 5 | --- |
| A | sc | 0,25 | 2 | 7,0 |
| A | sc | 2,5 | 4 | 7,0 |
| A | sc | 25,0 | 3 | 7,0 |
| Kontrolle | po | 0,0 | 1 | 7,0 |
| C | po | 0,25 | 1 | 8,0 |
| C | po | 2,5 | 4 | 8,0 |
| C | po | 25 | 4 | 7,0 |
| Kontrolle | po | 0,0 | 1 | 7,7 |
| Amantadin | po | 80,0 | 5 | --- |
| D | po | 0,25 | 4 | 8,0 |
| D | po | 2,5 | 5 | --- |
| D | po | 25,0 | 5 | --- |
| Kontrolle | sc | 0,0 | 0 | 7,4 |
| Amantadin | sc | 80,0 | 5 | --- |
| D | sc | 0,25 | 4 | 7,0 |
| D | sc | 2,5 | 2 | 8,0 |
| D | sc | 25,0 | 4 | 7,0 |

Fortsetzung Tabelle 3

Influenza A2

| Substanz | | Dosierung (mg/kg) | Überlebende | Mittlere Überlebenszeit (Tage) |
|---|---|---|---|---|
| Kontrolle | po | 0,0 | 2 | 7,3 |
| Amantadin | po | 80,0 | 5 | --- |
| E | po | 2,5 | 5 | --- |
| E | po | 25,0 | 5 | --- |
| E | po | 250,0 | 2 | 2,3 |
| Kontrolle | sc | 0,0 | 0 | 6,6 |
| Amantadin | sc | 80,0 | 5 | --- |
| E | sc | 2,5 | 3 | 7,0 |
| E | sc | 25,0 | 2 | 6,3 |
| E | sc | 250,0 | 0 | toxisch |
| Kontrolle | po | 0,0 | 2 | 6,7 |
| G | po | 0,25 | 5 | --- |
| G | po | 2,5 | 3 | 6,5 |
| G | po | 25 | 1 | 7,7 |
| Kontrolle | po | 0,0 | 2 | 6,7 |
| H | po | 0,25 | 3 | 5,5 |
| H | po | 2,5 | 4 | 8,0 |
| H | po | 25 | 3 | 8,5 |
| Kontrolle | po | 0,0 | 2 | 6,7 |
| I | po | 0,25 | 3 | 6,5 |
| I | po | 2,5 | 4 | 6,0 |
| I | po | 25 | 3 | 7,0 |
| Kontrolle | po | 0,0 | 2 | 6,7 |
| K | po | 0,25 | 4 | 6,0 |
| K | po | 2,5 | 2 | 7,0 |
| K | po | 25 | 3 | 6,5 |

Fortsetzung Tabelle 3

Influenza A2

| Substanz | | Dosierung (mg/kg) | Überlebende | Mittlere Überlebenszeit (Tage) |
|---|---|---|---|---|
| Kontrolle | po | 0,0 | 2 | 7,7 |
| Amantadin | po | 80,0 | 5 | --- |
| L | po | 0,25 | 4 | 8,0 |
| L | po | 2,5 | 3 | 6,0 |
| L | po | 25,0 | 5 | --- |
| Kontrolle | sc | 0,0 | 1 | 6,5 |
| Amantadin | sc | 80,0 | 5 | --- |
| L | sc | 0,25 | 3 | 9,5 |
| L | sc | 2,5 | 0 | 6,8 |
| L | sc | 25,0 | 4 | 10,0 |
| Kontrolle | po | 0,0 | 2 | 8,3 |
| Amantadin | po | 80,0 | 5 | --- |
| M | po | 0,25 | 4 | 7,0 |
| M | po | 2,5 | 4 | 7,0 |
| M | po | 25,0 | 5 | --- |
| Kontrolle | sc | 0,0 | 1 | 6,5 |
| M | sc | 0,25 | 5 | --- |
| M | sc | 2,5 | 3 | 7,0 |
| M | sc | 25 | 5 | --- |
| Kontrolle | po | 0,0 | 0 | 6,6 |
| N | po | 0,25 | 4 | 8,0 |
| N | po | 2,5 | 3 | 8,0 |
| N | po | 25 | 3 | 6,5 |
| Kontrolle | po | 0,0 | 0 | 7,2 |
| O | po | 0,25 | 5 | --- |
| O | po | 2,5 | 3 | 8,5 |
| O | po | 25 | 3 | 9,0 |

**Fortsetzung Tabelle 3**

Influenza A2

| Substanz | | Dosierung (mg/kg) | Überlebende | Mittlere Überlebenszeit (Tage) |
|---|---|---|---|---|
| Kontrolle | po | 0,0 | 1 | 7,0 |
| P | po | 0,25 | 3 | 8,5 |
| P | po | 2,5 | 4 | 7,0 |
| P | po | 25 | 3 | 6,5 |
| Kontrolle | sc | 0,0 | 0 | 6,4 |
| R | sc | 0,25 | 3 | 9,0 |
| R | sc | 2,5 | 4 | 7,0 |
| R | sc | 25 | 0 | 6,8 |
| Kontrolle | sc | 0,0 | 0 | 6,4 |
| T | sc | 0,25 | 3 | 7,0 |
| T | sc | 2,5 | 4 | 8,0 |
| T | sc | 25 | 4 | 7,0 |
| Kontrolle | sc | 0,0 | 1 | 8,5 |
| U | sc | 0,25 | 4 | 7,0 |
| U | sc | 2,5 | 4 | 7,0 |
| U | sc | 25 | 2 | 7,3 |
| Kontrolle | po | 0,0 | 1 | 7,0 |
| V | po | 0,25 | 4 | 7,0 |
| V | po | 2,5 | 3 | 9,0 |
| V | po | 25 | 3 | 7,5 |
| Kontrolle | po | 0,0 | 2 | 7,0 |
| W | po | 2,5 | 4 | 9,0 |
| W | po | 25 | 4 | 9,0 |
| W | po | 250 | 3 | 7,0 |

**Fortsetzung Tabelle 3**

Influenza A2

| Substanz | | Dosierung (mg/kg) | Überlebende | Mittlere Überlebens-zeit (Tage) |
|---|---|---|---|---|
| Kontrolle | po | 0,0 | 0 | 6,6 |
| X | po | 0,25 | 3 | 7,0 |
| X | po | 2,5 | 4 | 6,0 |
| Kontrolle | po | 0,0 | 2 | 8,3 |
| Y | po | 0,25 | 2 | 8,3 |
| Y | po | 2,5 | 2 | 6,7 |
| Y | po | 25 | 4 | 8,0 |
| Kontrolle | po | 0,0 | 0 | 7,4 |
| Z | po | 0,25 | 3 | 7,0 |
| Z | po | 2,5 | 4 | 7,0 |
| Z | po | 25 | 1 | 7,3 |
| Kontrolle | po | 0,0 | 0 | 7,4 |
| AB | po | 0,25 | 4 | 9,0 |
| AB | po | 2,5 | 4 | 6,0 |
| AB | po | 25 | 1 | 7,5 |
| Kontrolle | sc | 0,0 | 0 | 6,2 |
| AC | sc | 0,25 | 2 | 6,3 |
| AC | sc | 2,5 | 1 | 7,0 |
| AC | sc | 25 | 4 | 6,0 |
| Kontrolle | sc | 0,0 | 0 | 6,4 |
| AD | sc | 0,25 | 2 | 7,0 |
| AD | sc | 2,5 | 3 | 6,5 |
| AD | sc | 25 | 4 | 7,0 |
| Kontrolle | po | 0,0 | 2 | 7,7 |
| Amantadin | po | 80 | 5 | --- |
| AE | po | 0,25 | 2 | 7,0 |
| AE | po | 2,5 | 4 | 7,0 |
| AE | po | 25 | 5 | --- |

**Fortsetzung Tabelle 3**

Influenza A2

| Substanz | | Dosierung (mg/kg) | Überlebende | Mittlere Überlebenszeit (Tage) |
|---|---|---|---|---|
| Kontrolle | po | 0,0 | 0 | 7,2 |
| AF | po | 0,25 | 4 | 10,0 |
| AF | po | 2,5 | 5 | --- |
| AF | po | 25 | 0 | 7,2 |
| Kontrolle | po | 0,0 | 2 | 6,7 |
| AH | po | 0,25 | 4 | 9,0 |
| AH | po | 2,5 | 3 | 7,0 |
| AH | po | 25 | 2 | 8,0 |
| Kontrolle | po | 0,0 | 2 | 8,7 |
| AL | po | 0,25 | 0 | 8,0 |
| AL | po | 2,5 | 2 | 7,3 |
| AL | po | 25 | 5 | --- |

po = oral
sc = subkutan

## Beispiel 40

Labormäuse (NMRI, weiblich, Gewicht 20 bio 24 g) wurden mit Friend-Leukämie Virus-haltigem (FLV) Mäuseserum intravenös infiziert. 48 h nach der Infektion begann eine 10-tägige Behandlung. Die Mäuse wurden mit den in Tabelle 4 angegebenen Substanzen behandelt. Einmal pro Tag wurden die angegebenen Substanzen oral oder intraperitoneal verabreicht. 14 Tage nach Infektion wurden die Tiere durch zervicale Dislocation getötet und die Milzen wurden entnommen. Das Gewicht der Milzen wurde bestimmt. Als Meßparameter der therapeutischen Wirksamkeit wurde das Milzgewicht der behandelten Tiere mit dem der unbehandelten Kontrolltiere in Relation gesetzt.

Als Standardsubstanzen dienten Suramin und Azidothymidin (AZT). Die Wirkung der Substanzen ergibt sich aus Tabelle 4.

**Tabelle 4**

FLV

| Substanz | | Dosierung (mg/kg) | Überlebens-rate (%) | relatives Milz-gewicht (%) |
|---|---|---|---|---|
| Kontrolle | po | 0,0 | 100 | 12,36 |
| AZT | po | 15,5 | 100 | 3,26 |
| E | po | 16,5 | 100 | 6,94 |
| E | po | 77,5 | 100 | 8,54 |
| Kontrolle | ip | 0,0 | 100 | 8,80 |
| E | ip | 50,0 | 90 | 4,83 |
| Kontrolle | po | 0,0 | 100 | 12,36 |
| AZT | po | 15,5 | 100 | 3,26 |
| C | po | 16,0 | 100 | 6,5 |
| C | po | 75,0 | 100 | 6,47 |
| Kontrolle | ip | 0,0 | 100 | 8,08 |
| Suramin | ip | 50,0 | 100 | 6,70 |
| C | ip | 50,0 | 100 | 6,55 |
| Kontrolle | po | 0,0 | 100 | 10,50 |
| AZT | po | 14,5 | 100 | 1,81 |
| A | po | 15,0 | 100 | 6,93 |
| A | po | 73,5 | 100 | 6,15 |
| Kontrolle | ip | 0,0 | 100 | 10,50 |
| Suramin | ip | 50,0 | 100 | 6,17 |
| A | ip | 50,0 | 100 | 7,22 |

**Fortsetzung Tabelle 4**

FLV

| Substanz | | Dosierung (mg/kg) | Überlebens-rate (%) | relatives Milz-gewicht (%) |
|---|---|---|---|---|
| Kontrolle | ip | 0,0 | 100 | 10,42 |
| Suramin | ip | 50,0 | 60 | 4,12 |
| B | ip | 50,0 | 80 | 6,60 |
| Kontrolle | ip | 0,0 | 100 | 10,50 |
| Suramin | ip | 50,0 | 100 | 6,17 |
| M | ip | 50,0 | 90 | 7,46 |
| Kontrolle | ip | 0,0 | 100 | 8,52 |
| AZT | ip | 50 | 100 | 5,40 |
| O | ip | 30 | 90 | 5,06 |
| O | ip | 10 | 90 | 6,32 |
| Kontrolle | ip | 0,0 | 100 | 10,42 |
| Suzamin | ip | 50 | 60 | 4,12 |
| U | ip | 50 | 90 | 7,56 |
| Kontrolle | po | 0,0 | 100 | 11,36 |
| AZT | po | 18,0 | 100 | 6,42 |
| K | po | 15,5 | 90 | 9,02 |

po = oral
ip = intraperitoneal

## Patentansprüche

1. Verbindung der Formel I,

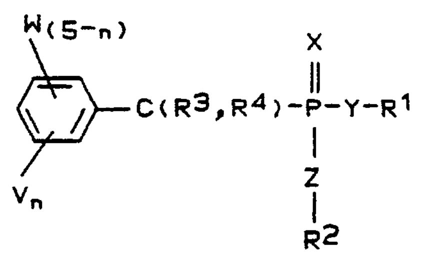

I

in der
V

für eine geradkettige oder verzweigte Alkylgruppe mit 1 bis 4 Kohlenstoffatomen, Fluor, Chlor, Brom, Jod oder Wasserstoff steht,

n

für eine ganze Zahl von 1 bis 5 steht,

W

für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine geradkettige oder verzweigte Alkoxygruppe mit 1 bis 10 Kohlenstoffatomen, eine Arylgruppe mit 6, 10 oder 14 Kohlenstoffatomen, eine Alkylgruppe mit 1 bis 4 Kohlenstoffatomen, ein oder mehrfach substituiert durch den Rest der Formel

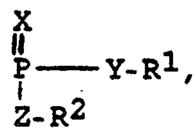

den Rest der Formel -COOR$^6$ oder Halogen, in der Halogen Chlor, Brom, Jod oder Fluor bedeutet, einen ankondensierten Aromaten, der durch eine Alkylgruppe mit 1 bis 4 Kohlenstoffatomen substituiert sein kann, Cyanid, Nitro oder Wasserstoff steht oder für den Rest der Formel Ia steht,

-COOR$^6$    Ia

wobei

R$^6$

eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff oder eine geradkettige oder verzweigte Halogenalkylgruppe mit 1 bis 20 Kohlenstoffatomen bedeutet, in der Halogen Chlor, Brom, Jod oder Fluor bedeutet,

R$^1$ und R$^2$,

die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff, eine geradkettige oder verzweigte Halogenalkylgruppe mit 1 bis 20 Kohlenstoffatomen stehen, in der Halogen Chlor, Brom, Jod oder Fluor bedeutet, oder R$^1$ und R$^2$ zusammen einen cyclischen Diester mit 2 bis 6 Kohlenstoffatomen im Ring bilden,

R$^3$ und R$^4$,

die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Aralkylgruppe mit 7 bis 20 Kohlenstoffatomen, eine geradkettige oder verzweigte Alkoxygruppe mit 1 bis 10 Kohlenstoffatomen, Wasserstoff, Fluor, Chlor, Brom oder Jod stehen oder für den Rest der Formel Ib stehen,

-COOR$^7$    Ib

wobei

R$^7$

eine geradkettige oder verzweigte Alkylgruppe mit 1 bis 4 Kohlenstoffatomen bedeutet und

X, Y und Z,

die gleich oder verschieden sein können, für Sauerstoff oder Schwefel stehen,

als Arzneimittel.

2. Verbindung der Formel I nach Anspruch 1,
in der

V
für Brom, Wasserstoff oder Methyl steht,
n
für eine ganze Zahl von 1 bis 5 steht,
W
für Methoxy oder Wasserstoff steht,
$R^1$ und $R^2$,
die gleich oder verschieden sein können, für eine geradkettige oder verzweigte Alkylgruppe mit 1 bis 10 Kohlenstoffatomen, eine Alkenyl- oder Alkinylgruppe mit 2 bis 10 Kohlenstoffatomen, eine Aralkylgruppe mit 7 bis 16 Kohlenstoffatomen oder Wasserstoff stehen,
$R^3$ und $R^4$,
die gleich oder verschieden sein können, für eine geradkettige oder verzweigte Alkylgruppe mit 1 bis 4 Kohlenstoffatomen, eine Alkenyl- oder Alkinylgruppe mit 2 bis 10 Kohlenstoffatomen, eine geradkettige oder verzweigte Alkoxygruppe mit 1 bis 4 Kohlenstoffatomen, Wasserstoff, Fluor, Chlor oder Brom stehen oder für den Rest der Formel Ib stehen,

$-COOR^7$ Ib.

wobei
$R^7$
eine geradkettige oder verzweigte Alkylgruppe mit 1 bis 4 Kohlenstoffatomen bedeutet und
X, Y und Z
für Sauerstoff steht,
als Arzneimittel.

3. Arzneimittel, dadurch gekennzeichnet, daß es eine wirksame Menge der Verbindung nach Anspruch 1 oder 2 neben üblichen Trägerstoffen, Hilfsstoffen und/oder Zusatzstoffen enthält.

4. Arzneimittel, dadurch gekennzeichnet, daß es wirksame Mengen einer oder mehrerer Verbindungen nach Anspruch 1 oder 2 enthält.

5. Arzneimittel nach Anspruch 4, dadurch gekennzeichnet, daß zusätzlich übliche Trägerstoffe, Hilfsstoffe und/oder Zusatzstoffe enthalten sind.

6. Arzneimittel nach einem oder mehreren der Ansprüche 1 bis 5 zur Behandlung von Erkrankungen, die durch DNA- oder RNA-Viren hervorgerufen werden.

7. Verwendung der Verbindung der Formel I,

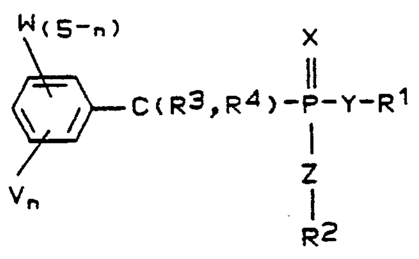

I

in der
V
für eine geradkettige oder verzweigte Alkylgruppe mit 1 bis 4 Kohlenstoffatomen, Fluor, Chlor, Brom, Jod oder Wasserstoff steht, n
für eine ganze Zahl von 1 bis 5 steht,
W
für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine geradkettige oder

verzweigte Alkoxygruppe mit 1 bis 10 Kohlenstoffatomen, eine Arylgruppe mit 6, 10 oder 14 Kohlenstoffatomen, eine Alkylgruppe mit 1 bis 4 Kohlenstoffatomen, ein oder mehrfach substituiert durch den Rest der Formel

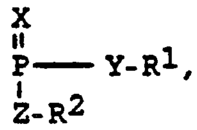

den Rest der Formel -$COOR^6$ oder Halogen, in der Halogen Chlor, Brom, Jod oder Fluor bedeutet, einen ankondensierten Aromaten, der durch eine Alkylgruppe mit 1 bis 4 Kohlenstoffatomen substituiert sein kann, Cyanid, Nitro oder Wasserstoff steht oder für den Rest der Formel Ia steht,

-$COOR^6$ Ia

wobei
$R^6$
eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff oder eine geradkettige oder verzweigte Halogenalkylgruppe mit 1 bis 20 Kohlenstoffatomen bedeutet, in der Halogen Chlor, Brom, Jod oder Fluor bedeutet,
$R^1$ und $R^2$,
die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Natrium, Kalium, Calcium, Magnesium, Aluminium, Lithium, Ammonium, Triethylammonium, Wasserstoff, eine geradkettige oder verzweigte Halogenalkylgruppe mit 1 bis 20 Kohlenstoffatomen stehen, in der Halogen Chlor, Brom, Jod oder Fluor bedeutet, oder $R^1$ und $R^2$ zusammen einen cyclischen Diester mit 2 bis 6 Kohlenstoffatomen im Ring bilden,
$R^3$ und $R^4$,
die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Aralkylgruppe mit 7 bis 20 Kohlenstoffatomen, eine geradkettige oder verzweigte Alkoxygruppe mit 1 bis 10 Kohlenstoffatomen, Wasserstoff, Fluor, Chlor, Brom oder Jod stehen oder für den Rest der Formel Ib stehen,

-$COOR^7$ Ib

wobei
$R^7$
eine geradkettige oder verzweigte Alkylgruppe mit 1 bis 4 Kohlenstoffatomen bedeutet und
X, Y und Z,
die gleich oder verschieden sein können, für Sauerstoff oder Schwefel stehen,
zur Herstellung eines Arzneimittels zur Behandlung von Erkrankungen, die durch DNA- oder RNA-Viren hervorgerufen werden.

**8.** Verwendung der Verbindung der Formel I nach Anspruch 7,
in der
V
für Brom, Wasserstoff oder Methyl steht,
n
für eine ganze Zahl von 1 bis 5 steht,
W
für Methoxy oder Wasserstoff steht,
$R^1$ und $R^2$,

die gleich oder verschieden sein können, für eine geradkettige oder verzweigte Alkylgruppe mit 1 bis 10 Kohlenstoffatomen, eine Alkenyl- oder Alkinylgruppe mit 2 bis 10 Kohlenstoffatomen, eine Aralkylgruppe mit 7 bis 16 Kohlenstoffatomen oder Wasserstoff stehen,

$R^3$ und $R^4$,

die gleich oder verschieden sein können, für eine geradkettige oder verzweigte Alkylgruppe mit 1 bis 4 Kohlenstoffatomen, eine Alkenyl- oder Alkinylgruppe mit 2 bis 10 Kohlenstoffatomen, eine geradkettige oder verzweigte Alkoxygruppe mit 1 bis 4 Kohlenstoffatomen, Wasserstoff, Fluor, Chlor oder Brom stehen oder für den Rest der Formel Ib stehen,

$-COOR^7$ Ib

wobei

$R^7$

eine geradkettige oder verzweigte Alkylgruppe mit 1 bis 4 Kohlenstoffatomen bedeutet und

X, Y und Z,

für Sauerstoff steht,

zur Herstellung eines Arzneimittels zur Behandlung von Erkrankungen, die durch DNA- oder RNA-Viren hervorgerufen werden.

**9.** Verfahren zur Herstellung eines Arzneimittels, dadurch gekennzeichnet, daß eine wirksame Menge einer oder mehrerer Verbindungen nach Anspruch 7 in eine geeignete Darreichungsform überführt wird.

10. Verfahren zur Behandlung von durch DNA oder RNA Viren verursachten Krankheiten, dadurch gekennzeichnet, daß eine wirksame Menge einer oder mehrerer Verbindungen gemäß den Ansprüchen 7 oder 8 verabreicht wird.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 91101097
- Seite 1 -

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-2022589 (STERLING DRUG)<br>* das ganze Dokument * | 1-9 | A61K31/66<br>C07F9/40 |
| A | EP-A-192327 (THE WELLCOME FOUNDATION)<br>* das ganze Dokument * | 1-9 | |
| A | GB-A-2165153 (SEUREF)<br>* Zusammenfassung * | 1-9 | |
| A | FR-B-2358882 (F. HOFFMANN-LA ROCHE)<br>* Seite 3, Zeile 34 * | 1-9 | |
| A | FR-A-2449097 (SANDOZ)<br>* das ganze Dokument * | 1-9 | |
| | ... | | |

RECHERCHIERTE SACHGEBIETE (Int. Cl.5)

A61K
C07F

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: -
Nicht recherchierte Patentansprüche: 10
Grund für die Beschränkung der Recherche:

Artikel 52(4) EPÜ

| Recherchenort | Abschlußdatum der Recherche | Prüfer |
|---|---|---|
| Berlin | 24.04.1991 | P. AVEDIKIAN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82

Europäisches Patentamt

# EUROPÄISCHER TEILRECHERCHENBERICHT

Nummer der Anmeldung
EP 91101097
- Seite 2 -

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich, der maßgeblichen Teile | betrifft Anspruch | |
| A | NUCLEOSIDES & NUCLEOTIDES<br>Band 8, Nr. 5&6, 1989, Seiten 833-836, New York, US; R.W. SIDWELL et al.: "Effect of phosphonic acid analogs of acyclovir and ganciclovir on in vitro cytomegalovirus infections"<br>* das ganze Dokument * | 1-9 | |
| A | CHEMICAL ABSTRACTS<br>Band 111, Nr. 5, 31. Juli 1989, Seite 19, Zusammenfassung Nr. 33158w, Columbus, Ohio, US; N.D. L'VOV et al.: "Antiherpes activity of phosphonic acid derivatives and their combinations with interferon inducers on the model of eye herpes" & Vopr. Virusol. 1989, Band 34, Nr. 2, Seiten 186-192<br>* Zusammenfassung * | 1-9 | RECHERCHIERTE SACHGEBIETE (Int.Cl.5) |
| A | BIOLOGICAL ABSTRACT<br>Nr. 86128890; P.M. MALYKHIN et al.: "The effectiveness of domestic synthetic preparations in protecting strawberries from viral infections" & Izv. Timiryazev. S-KH Akad. 0 1988, Nr. 2, Seiten 110,111 | 1-9 | |